# EUROPÄISCHE PATENTSCHRIFT

(11) **EP 0 317 883 B1**
(45) Veröffentlichungstag und Bekanntmachung des Hinweises auf die Patenterteilung: **16.06.1993**
(21) Anmeldenummer: 88119070.6
(22) Anmeldetag: 17.11.1988
(51) Int. Cl.: C07C 43/29, C07C 43/295

(54) **Teilfluorierte Diphenylether, Verfahren zu ihrer Herstellung und ihre Verwendung**
Partially fluorinated diphenyl ethers, process for their preparation and their use
Ethers diphényl partiellement fluorés, procédé pour leur préparation et leur utilisation

(30) Priorität: 24.11.1987 DE 3739795
(43) Veröffentlichungstag der Anmeldung: 31.05.1989
(73) Patentinhaber: HOECHST AKTIENGESELLSCHAFT, 65926 Frankfurt am Main (DE)
(72) Erfinder: Siegemund, Günter, Dr., D-6238 Hofheim am Taunus (DE)

(56) Entgegenhaltungen:
- EP-A- 0 317 940
- EP-A- 0 317 942
- WO-A-86/01504
- US-A- 3 310 573
- US-A- 3 355 500

## Beschreibung

Die Erfindung betrifft neue, teilfluorierte Diphenylether, Verfahren zu ihrer Herstellung in Gegenwart von Fluorwasserstoff und ihre Verwendung als Zwischenprodukte für Bausteine teilfluorierter Polykondensate.

Teilfluorierte Diphenylether sind bekannt. So beschreibt das US- A 3,355,500 die Herstellung von 4,4'-Bis-(hexafluor-2-hydroxy-2-propyl)-diphenylether. Diarylfluorverbindungen, bei denen zwei substituierte Phenylenkerne über eine Hexafluorisopropylidenbrücke verbunden sind, sind aus US-A 3,310,573 und aus I.L. Knunyants et al., Izv. Akad. Nauk. SSSR, Otdel. Khim. Nauk. 4, 668-692 (1960); englische Ausgabe S. 647-653 (1960) bekannt. Die Umsetzung der bekannten Dihydroxyverbindungen in Gegenwart von Fluorwasserstoff zu höhermolekularen Produkten, die über zwei Hexafluorisopropylidenbrücken verbunden sind, kann dem Stand der Technik jedoch nicht entnommen werden.

Gegenstand der Erfindung sind Verbindungen der Formel 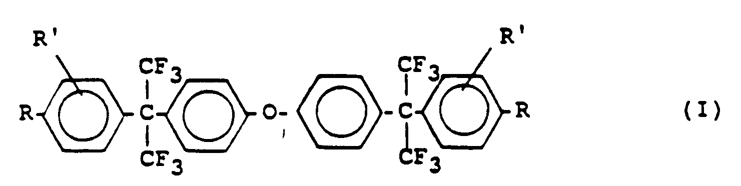
in der unabhängig voneinander R gleich OH oder niederes Alkyl mit 1 bis 4 C-Atomen und R' gleich Wasserstoff oder niederes Alkyl mit 1 bis 4 C-Atomen ist, wobei das Alkyl vorzugsweise CH₃ ist, ein Verfahren zu ihrer Herstellung und ihre Verwendung. In der Formel I befindet sich der Rest R' vorzugsweise in o-Stellung zum Rest R.

Die Verbindungen gemäß Erfindung können im allgemeinen nach zwei Verfahren hergestellt werden, und zwar a) durch Kondensation von einem Mol eines Dicarbinols a₁) der Formel 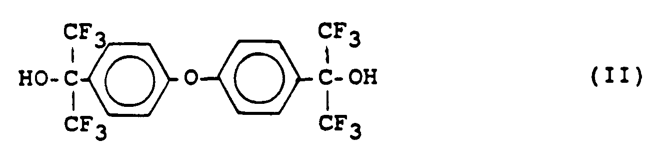
mit mindestens 2 Mol eines aromatischen substituierten Kohlenwasserstoffes a₂) der Formel 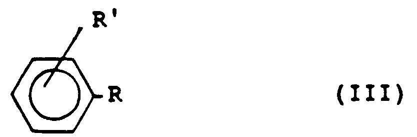
oder b) durch Kondensation von mindestens 2 Mol einer Verbindung b₁) der Formel 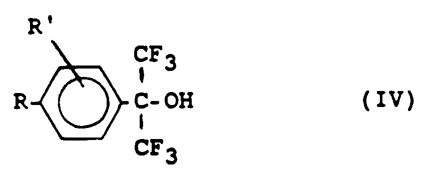
mit einem Mol Diphenylether (V) b₂) in Gegenwart von Fluorwasserstoff. Beispiele für aromatische substituierte Kohlenwasserstoffe III sind Phenylenverbindungen, die mit OH und/oder Alkylresten mit 1 bis 4 C-Atomen substituiert sind, wie Phenol, Toluol, die verschiedenen Xylole und Kresole. Verbindungen der Formel (II), die bei Verfahren a) eingesetzt werden können, sind in der US-A 3,355,500 beschrieben.

Verbindungen der Formel (IV), die gemäß Verfahren b) zu den erfindungsgemäßen Verbindungen umgesetzt werden, sind ebenfalls bekannt und in J.O.C. 30, S. 998-1001 (1965), beschrieben. Diese Verbindungen werden mit Diphenylether zur Reaktion gebracht.

Die Reaktionstemperaturen bei den Verfahren a) und b) liegen zwischen 80 und 180°C, vorzugsweise zwischen 100 und 170°C.

Die Reaktionszeiten betragen im allgemeinen 24 bis 90, vorzugsweise 65 bis 90 Stunden.

Das Molverhältnis der eingesetzten Reaktionspartner wird bestimmt bei Verfahren a) durch das Verhältnis der Verbindungen (II) : Verbindungen (III), sowie bei Verfahren b) durch das Verhältnis des Diphenylethers : Verbindungen (IV), es beträgt in beiden Fällen im allgemeinen mindestens 1:2, vorzugsweise 1 :(2,2 bis 4) .

Der Anteil an Fluorwasserstoff (HF), der bei der Reaktion zur Herstellung der erfindungsgemäßen nötig ist, wird bei Verfahren a) auf die Verbindung (II) bezogen und liegt im allgemeinen im Molverhältnis 1 :(7 bis 25) , vorzugsweise 1 :(10 bis 20).Bei dem Verfahren b) beträgt das Molverhältnis der Verbindungen (IV) : (HF) im allgemeinen 1 : (6 bis 15), vorzugsweise 1 : (8 bis 13).

Zur Aufarbeitung des Reaktionsgutes wird im allgemeinen der Fluorwasserstoff nach Beendigung der Reaktion bei ca. 80°C aus dem Reaktor abgegast und der verbleibende Rückstand, gegebenenfalls nach Verdünnen mit einem organischen Lösungsmittel, z.B. bei Raumtemperatur, dem Reaktor entnommen. Das erhaltene Rohgemisch wird mit Wasser vermischt, gewaschen und abgetrennt. Zur weiteren Reinigung kann das Reaktionsgut einer Umkristallisation aus einem organischen Lösungsmittel mit oder ohne Vorbehandlung mit Aktivkohle unterworfen werden, oder es erfolgt ein Ausrühren in organischen Lösungsmitteln, vorzugweise in Chlorpropan oder Methanol. Als Lösungsmittel, die bei der Aufarbeitung eingesetzt werden können, eignen sich aliphatische Kohlenwasserstoffe mit 5 bis 10 C-Atomen, aliphatische Monoalkohole mit 1 bis 4 C-Atomen im Alkylrest und ein- oder mehrfach chlorierte aliphatische Kohlenwasserstoffe mit 1 bis 4 C-Atomen im Alkylrest. Beispiele hierfür sind n-Hexan, n-Heptan, Methanol, Ethanol, die verschiedenen Propanole und Butanole, sowie Chlorpropan und Di- und Trichlormethan. Im allgemeinen fallen die gereinigten Produkte als farblose Kristallisate an.

Spezielle neue teilfluorierte Diphenylether, die im Rahmen der Erfindung liegen, sind:
4,4'-Bis[2-(4-hydroxyphenyl)-hexafluor-isopropyl]-diphenylether,
4,4'-Bis[2-(4-methylphenyl)-hexafluor-isopropyl]-diphenylether,
4,4'-Bis[2-(3,4-dimethylphenyl)-hexafluor-isopropyl]-diphenylether.

Die neuen Verbindungen können zur Herstellung von Synthesebausteinen für teilfluorierte Polykondensate, z.B. Polyester, Polyamide und -imide verwendet werden, wie sie in den Patentanmeldungen vom selben Tage....... EP-A 0317884 "Teilfluorierte Carbonsäuren, sowie Derivate davon, Verfahren zu ihrer Herstellung und Verwendung" und .... EP-A 0317882 "Fluorhaltige Verbindungen auf Basis von 4,4'-Bis-[2-(4-hydroxyphenyl)hexafluorisopropyl]-diphenylether, Verfahren zu ihrer Herstellung und ihre Verwendung" beschrieben worden sind.

In den folgenden Beispielen bedeutet VA-Stahl Chrom-Nickel-Stahl.

### Beispiele

### 1) 4,4'-Bis[2-(4-hydroxyphenyl)-hexafluor-isopropyl]-diphenylether

a) In einem 1 l Rührautaklaven aus VA-Stahl wurden 251 g 4,4'-Bis[hexafluor-2-hydroxy-2-propyl]-diphenylether, 94 g Phenol und 180 g wasserfreier Fluorwasserstoff vereinigt und 72 Stunden bei 120°C gerührt. Nach Abgasen des Fluorwasserstoffs wurde das zum Teil feste Produkt in Ethanol aufgeschlämmt und dem Autoklaven entnommen. Durch Erhitzen in Gegenwart von 30 g Aktivkohle wurde das Rohprodukt in Lösung gebracht, aus der nach Abkühlen 278 g eines kristallinen Produktes 4,4'-Bis[2-(4-hydroxyphenyl)-hexafluorisopropyl]-diphenylether (Ausbeute: 85 %) ausfielen, das nach Waschen mit Wasser und Trocknen einen Schmelzpunkt von 179 bis 180°C besaß.

| Analyse für C₃₀H₁₈F₁₂O₃: | | | |
|---|---|---|---|
| Berechnet: | C 55,04 % | H 2,75 % | F 34,86 % |
| Gefunden: | C 55,00 % | H 2,60 % | F 35,10 % |

b) Ein 2 l VA-Stahlreaktor wurde mit 68 g 4,4'-Bis-[hexafluor-2-hydroxy-2-propyl]-diphenylether, 282 g Phenol und 450 g wasserfreiem Fluorwasserstoff gefüllt. Die Kondensationsreaktion wurde 66 Stunden bei 140°C unter Rühren durchgeführt. Nach Absenken der Temperatur auf 80°C wurde der Fluorwasserstoff abgegast. Anschließend wurde das feste Produkt dem Autoklaven entnommen, gut mit Wasser gewaschen und mit CaCl₂ getrocknet. Zur Reinigung wurde das Produkt mehrere Stunden in 1,5 l Chlorpropan aufgeschlämmt und gerührt. Nach Abtrennen von dem Lösungsmittel und Trocknen wurden 717 g kristallines Produkt (Ausbeute: 81,2 %) erhalten, das laut Gaschromatogramm eine Reinheit von 99,1 % besitzt.
Schmelzpunkt: 179 bis 180°C.
c) In einem 1 l VA-Stahlautoklaven mit Rührer wurden 286 g 2-(4-Hydroxyphenyl)hexafluorpropanol-2 und 85 g Diphenylether vorgelegt. 200 g wasserfreier Fluorwasserstoff wurden dann in den verschlossenen Autoklaven gepumpt. Die Reaktion wurde bei 120°C 65 Stunden lang durchgeführt. Anschließend wurde der Fluorwasserstoff bei 80°C abgegast, der feste Rückstand mit Wasser aufgeschlämmt und aus dem Autoklaven gespült. Nach Waschen mit Wasser wurde das Produkt mit Aktivkohle und Ethanol versetzt, 2 Stunden erhitzt, filtriert und abgekühlt. Ausbeute 154 g ≙ 47 %.
Schmelzpunkt: 179 bis 180°C.

### 2) 4,4'-Bis[2-(4-methylphenyl)-hexafluor-isopropyl]-diphenylether

a) 825 g 2-(4-Methylphenyl)hexafluorpropanol-2, 256 g Diphenylether und 540 g wasserfreier Fluorwasserstoff wurden in einem 2 l VA-Stahlautoklaven 65 Stunden unter Rühren auf 170°C erwärmt. Bei 80°C wurde anschließend der Fluorwasserstoff abgegast; auf den erkalteten Rückstand wurden 500 ml n-Hexan gegeben, das Gemisch aufgerührt und aus dem Autoklaven herausgesaugt. Die organische Phase wurde zweimal mit Wasser gewaschen, das n-Hexan abgezogen und der Rückstand (775 g) mit 1,5 l Methanol ausgerührt. Das feste Produkt (587 g oder 60,2 % Ausbeute) wurde vom Methanol abgetrennt und getrocknet. Schmelzpunkt: 89 bis 90,5°C.

| Analyse für C₃₂H₂₂F₁₂O: | | | |
|---|---|---|---|
| Berechnet: | C 59,08 % | H 3,38 % | F 35,08 % |
| Gefunden: | C 59,50 % | H 3,35 % | F 35,10 % |

b) In einem 1 l Stahlautoklaven mit Rührer wurden 387 g 2-(4-Methylphenyl)hexafluorpropanol-2, 128 g Diphenylether und 270 g wasserfreier Fluorwasserstoff vereinigt und 64 Stunden bei 150°C gerührt. Bei 80°C wurde anschließend der Fluorwasserstoff abgegast, das Produkt aus dem Autoklaven herausgesaugt und mit 200 ml Methylenchlorid verdünnt. Die organische Lösung wurde zweimal mit Wasser gewaschen, über CaCl₂ getrocknet und destillativ eingeengt. Es verblieben 401 g, die,in 400 ml n-Hexan gelöst, durch eine Fritte unter Verwendung eines Filtrierhilfsmittels gesaugt und vom Lösungsmittel durch Destillation getrennt wurden. Der Rückstand (385 g oder 79 % Ausbeute) wurde aus 400 ml Ethanol umkristallisiert.
Schmelzpunkt: 89 bis 90°C.

### 3) 4,4'-Bis[2-(3,4-dimethylphenyl)-hexafluor-isopropyl]-diphenylether

a) In einem 2 l VA-Autoklaven wurden 256 g Diphenylether und 812 g 2-(3,4-Dimethylphenyl)-hexafluorpropanol-2 vorgelegt und 540 g wasserfreier Fluorwasserstoff eingepumpt. Anschließend wurde das Reaktionsgemisch unter Rühren 65 Stunden bei 150°C gehalten. Nach Abdampfen des Fluorwasserstoffs bei 80°C wurde das Produkt auf Raumtemperatur abgekühlt, mit 250 ml n-Hexan versetzt und dem Autoklaven entnommen. Nach kurzem Erhitzen am Rückfluß kristallisierten aus der n-Hexanlösung beim Abkühlen 466 g Produkt aus, die nach Umkristallisieren aus Ethanol einen Schmelzpunkt von 140 bis 141°C haben. Ausbeute: 45,8 %.

| Analyse für C₃₄H₂₆F₁₂O: | | | |
|---|---|---|---|
| Berechnet: | C 60,18 % | H 3,83 % | F 33,63 % |
| Gefunden: | C 60,15 % | H 3,80 % | F 33,70 % |

b) In einem 2 l VA-Autoklaven wurden 312 g 4,4'-Bis-(hexafluor-2-hydroxy-2-propyl)diphenylether und 268 g o-Xylol eingefüllt und anschließend in den verschlossenen Autoklaven 300 g wasserfreier Fluorwasserstoff eingepumpt. Das Reaktionsgemisch wurde 87 Stunden bei 120°C gerührt. Nach Abgasen des Fluorwasserstoffs bei 80°C wurde das auf Raumtemperatur abgekühlte Produkt dem Autoklaven entnommen und auf Eiswasser gegeben. Die organische Phase wurde mit 200 ml Dichlormethan verdünnt, die Lösung über CaCl₂ getrocknet. Nach Abdestillieren von Methylenchlorid und überschüssigem o-Xylol verblieben 358 g Rückstand, der nach Behandeln mit 35 g Aktivkohle und Umkristallisieren aus 400 ml Chloroform einen Schmelzpunkt von 139 bis 141°C zeigte. Ausbeute: 85,2 %.

## Patentansprüche

1. Verbindung der Formel 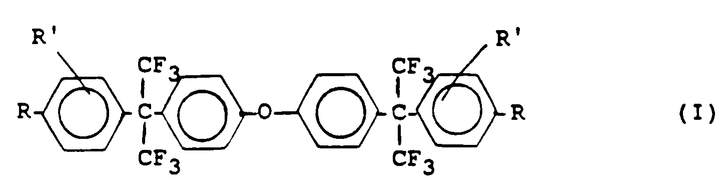 in der unabhängig voneinander R gleich OH oder niederes Alkyl mit 1 bis 4 C-Atomen, R' gleich Wasserstoff oder niederes Alkyl mit 1 bis 4 C-Atomen ist.

2. 4,4'-Bis[2-(2-(4-hydroxyphenyl)-hexafluor-isopropyl]-diphenylether.

3. 4,4'-Bis[2-(4-methylphenyl)-hexafluor-isopropyl]-diphenylether.

4. 4,4'-Bis[2-(3,4-dimethylphenyl)hexafluor-isopropyl]-diphenylether.

5. Verfahren zur Herstellung einer Verbindung der Formel 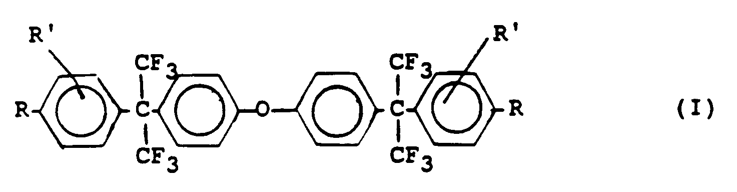 dadurch gekennzeichnet, daß
a) ein Dicarbinol a₁) der Formel 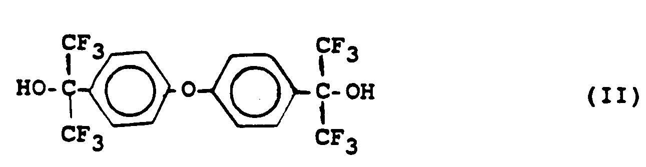 mit einem aromatischen Kohlenwasserstoff a₂) der Formel 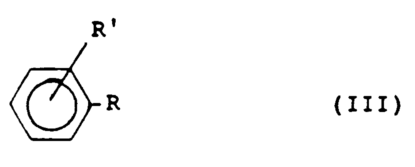 oder
b) eine Fluorverbindung b₁) der Formel 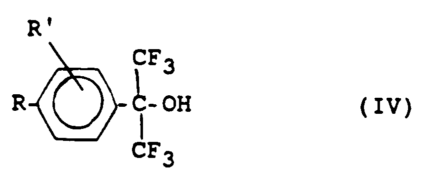 mit Diphenylether (V) b₂)
-in den Formeln haben R, R' die in Formel (I) genannte Bedeutung -,in Gegenwart von Fluorwasserstoff bei Temperaturen zwischen 80 und 180°C zur Reaktion gebracht werden, der Fluorwasserstoff nach der Reaktion entfernt und das Reaktionsprodukt aufgearbeitet wird.

6. Ausführungsform nach Anspruch 1 oder 5, dadurch gekennzeichnet, daß niederes Alkyl die Gruppe -CH₃ ist.

7. Ausführungsform nach Anspruch 1, 5 oder 6, dadurch gekennzeichnet, daß R' in ortho-Stellung zum Rest R steht.

8. Verfahren nach einem oder mehreren der Ansprüche 5 bis 7, dadurch gekennzeichnet, daß die Reaktionspartner im Verhältnis von 1 zu mindestens 2, vorzugsweise 1:(2,2 bis 4), beim Verfahren a) bezogen auf das Verhältnis von (II) zu (III) und beim Verfahren b) bezogen auf das Verhältnis von (V) zu (IV) eingesetzt werden.

9. Verfahren nach einem oder mehreren der Ansprüche 5 bis 8, dadurch gekennzeichnet, daß das Molverhältnis von Fluorwasserstoff zu Verbindung (II)(7 bis 25): 1, vorzugsweise (10 bis 20) : 1 und dasjenige zu Verbindung (IV)(6 bis 15): 1, vorzugsweise (8 bis 13): 1 ist.

10. Verfahren nach einem oder mehreren der Ansprüche 5 bis 9, dadurch gekennzeichnet, daß die Reaktion bei 100 bis 170°C durchgeführt wird.

11. Verfahren nach einem oder mehreren der Ansprüche 5 bis 10, dadurch gekennzeichnet, daß die Reaktion in 24 bis 90 Stunden durchgeführt wird.

12. Verfahren nach einem oder mehreren der Ansprüche 5 bis 11, dadurch gekennzeichnet, daß das Reaktionsprodukt, gegebenenfalls nach Verdünnung mit einem organischen Lösungsmittel, mit Wasser behandelt und anschließend aus einem organischen Lösungsmittel umkristallisiert oder ausgerührt wird.

13. Verfahren nach Anspruch 12, dadurch gekennzeichnet, daß mindestens ein Lösungsmittel aus der Gruppe aliphatische Kohlenwasserstoffe mit 5 bis 10 C-Atomen, aliphatische Monoalkohole mit 1 bis 4 C-Atomen im Alkylrest und ein- oder mehrfach chlorierte aliphatische Kohlenwasserstoffe mit 1 bis 4 C-Atomen im Alkylrest eingesetzt wird, wie n-Hexan, n-Heptan, Methanol, Ethanol, die verschiedenen Propanole und Butanole, sowie Chlorpropan und Di- und Trichlormethan.

14. Verwendung der Verbindung gemäß Anspruch 1 zur Herstellung von teilfluorierten Polykondensaten, insbesondere von teilfluorierten Polyestern, Polyamiden und Polyimiden.

## Claims

1. A compound of the formula 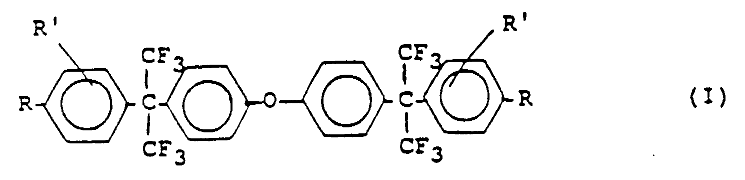 in which independently of one another R is equal to OH or lower alkyl having 1 to 4 carbon atoms, R' is equal to hydrogen or lower alkyl having 1 to 4 carbon atoms.

2. 4,4'-Bis[2-(4-hydroryphenyl)hexafluoroisopropyl]-diphenyl ether.

3. 4,4'-Bis[2-(4-methylphenyl)hexafluoroisopropyl]-diphenyl ether.

4. 4,4'-Bis-[2-(3,4-dimethylphenyl)hexafluoroisopropyl]-diphenyl ether.

5. A process for the preparation of a compound of the formula 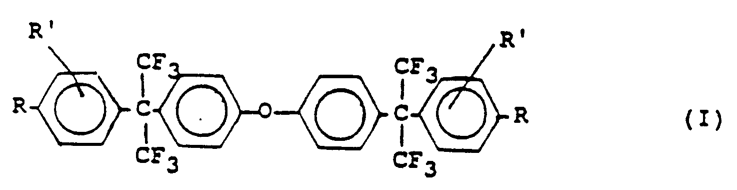 which comprises reacting
a) a dicarbinol a₁) of the formula 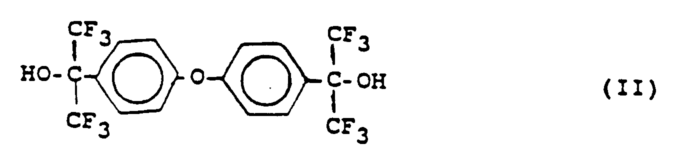 with an aromatic hydrocarbon a₂) of the formula 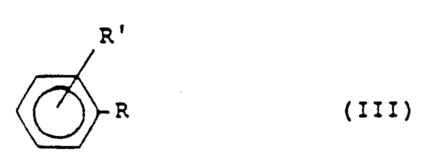 or
b) a fluoro compound b₁) of the formula 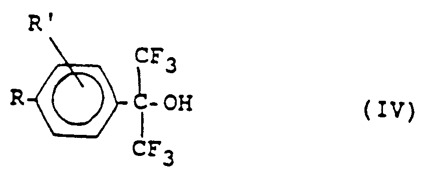 with diphenyl ether (V) b₂),
- R,R' in the formulae having the meaning mentioned in formula (I) - in the presence of hydrogen fluoride at temperatures between 80 and 180°C, removing the hydrogen fluoride after the reaction and working up the reaction product.

6. The embodiment as claimed in claim 1 or 5, wherein lower alkyl is the -CH₃ group.

7. The embodiment as claimed in claim 1, 5 or 6, wherein R' is in the ortho-position with respect to the radical R.

8. The process as claimed in one or more of claims 5 to 7, wherein the reactants are used in the ratio of 1 to at least 2, preferably 1 : (2.2 to 4), in process a) relative to the ratio of (II) to (III) and in process b) relative to the ratio of (V) to (IV).

9. The process as claimed in one or more of claims 5 to 8, wherein the molar ratio of hydrogen fluoride to compound (II) is (7 to 25) : 1, preferably (10 to 20) : 1 and that to compound (IV) is (6 to 15) : 1, preferably (8 to 13) : 1.

10. The process as claimed in one or more of claims 5 to 9, wherein the reaction is carried out at 100 to 170°C.

11. The process as claimed in one or more of claims 5 to 10, wherein the reaction is carried out in 24 to 90 hours.

12. The process as claimed in one or more of claims 5 to 11, wherein the reaction product, if necessary after dilution with an organic solvent, is treated with water and then recrystallized from an organic solvent or stirred up therein.

13. The process as claimed in claim 12, wherein at least one solvent of the group consisting of aliphatic hydrocarbons having 5 to 10 carbon atoms, aliphatic monoalcohols having 1 to 4 carbon atoms in the alkyl radical and mono- or polychlorinated aliphatic hydrocarbons having 1 to 4 carbon atoms in the alkyl radical is used, such as n-hexane, n-heptane, methanol, ethanol, the various propanols and butanols, and chloropropane and di- and trichloromethane.

14. Use of the compound as claimed in claim 1 for the preparation of partially fluorinated polycondensates, in particularly of partially fluorinated polyesters, polyamides and polyimides.

## Revendications

1. Composé de formule : 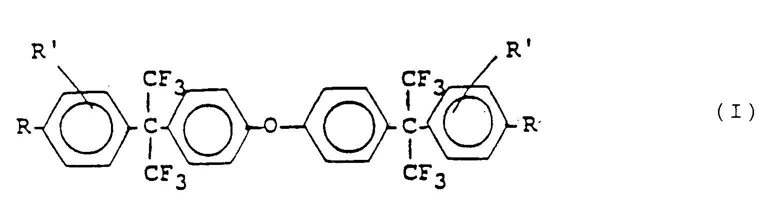 dans laquelle les radicaux R, indépendamment l'un de l'autre, sont chacun OH ou un radical alkyle inférieur ayant de 1 à 4 atomes de carbone, et les radicaux R', indépendamment l'un de l'autre, sont chacun un hydrogène ou un radical alkyle ayant de 1 à 4 atomes de carbone.

2. Oxyde de bis[(hydroxy-4 phényl)-2 hexafluoroisopropyl]-4,4' diphényle

3. Oxyde de bis[(méthyl-4 phényl)-2 hexafluoroisopropyl]-4,4' diphényle

4. Oxyde de bis[(diméthyl-3,4 phényl)-2 hexafluoroisopropyl]-4,4' diphényle

5. Procédé pour préparer un compose de formule : 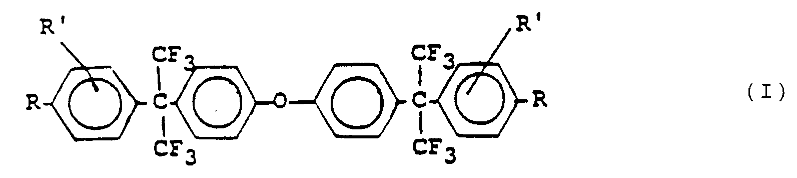 caractérise en ce qu'on fait réagir en présence d'acide fluorhydrique à des températures de 80 à 180°C
a) un dicarbinol a₁) de formule : 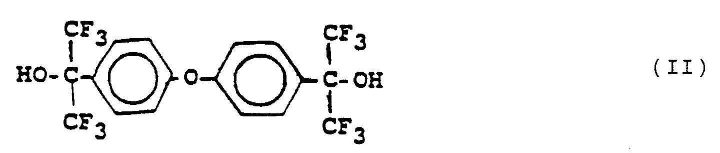 avec un hydrocarbure aromatique a₂) de formule : 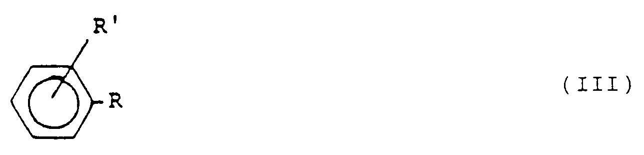 ou
b) un composé fluoré b₁) de formule : 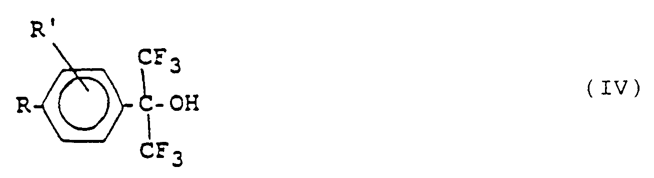 avec de l'oxyde de diphényle (V) b₂),
- dans les formules, R et R' ont les significations données pour la formule (I) -, on chasse l'acide fluorhydrique après la réaction et on traite le produit de la réaction.

6. Forme de réalisation selon la revendication 1 ou 5, caractérisée en ce que le radical alkyle inférieur est le groupe -CH₃.

7. Forme de réalisation selon la revendication 1, 5 ou 6, caractérisée en ce que R' se trouve en position ortho par rapport au radical R.

8. Procédé selon l'une ou plusieurs des revendications 5 à 7, caractérisé en ce que les substances participant à la réaction sont utilisées selon un rapport d'au moins 2, de préférence de 1:(2,2 à 4), le rapport étant celui des composés (II) et (III) dans le procédé a) et le rapport des composés (V) et (IV) dans le procédé b).

9. Procédé selon l'une ou plusieurs des revendications 5 à 8, caractérisé en ce que le rapport en moles de l'acide fluorhydrique au composé (II) est de (7 à 25):1 et de préférence de (10 à 20):1, et que le rapport en moles de l'acide fluorhydrique au composé (IV) est de (6 à 15):1 et de préférence de (8 à 13):1.

10. Procédé selon l'une ou plusieurs des revendications 5 à 9, caractérisé en ce que la réaction est mise en oeuvre à des températures de 100 à 170°C.

11. Procédé selon l'une ou plusieurs des revendications 5 à 10, caractérisé en ce que la réaction est mise en oeuvre en 24 à 90 heures.

12. Procédé selon l'une ou plusieurs des revendications 5 à 11, caractérisé en ce que le produit de la réaction, éventuellement après dilution par un solvant organique, est traité à l'eau puis recristallisé ou délayé dans un solvant organique.

13. Procédé selon la revendication 12, caractérisé en ce qu'on utilise au moins un solvant choisi parmi l'ensemble comprenant les hydrocarbures aliphatiques ayant de 5 à 10 atomes de carbone, à des monoalcools aliphatiques ayant de 1 à 4 atomes de carbone dans le résidu alkyle et à des hydrocarbures aliphatiques une ou plusieurs fois chlorés ayant de 1 à 4 atomes de carbone dans le résidu alkyle, tels que le n-hexane, le n-heptane, le méthanol, l'éthanol, les différents propanols et butanols, ainsi que le chloropropane et le diet le trichlorométhane.

14. Utilisation du composé selon la revendication 1 pour produire des produits de polycondensation partiellement fluorés, en particulier des polyesters, des polyamides et des polyimides partiellement fluorés.
